# EUROPEAN PATENT APPLICATION

(11) **EP 2 008 673 A2**
(43) Date of publication of application: **31.12.2008**
(21) Application number: 07017062.6
(22) Date of filing: 18.09.2007
(51) Int. Cl.: A61L 27/36, A61L 27/50, A61L 2/08

(54) **Method of irradiation using process of adding vitamin c**

(30) Priority: 26.06.2007 KR 20070063240
(71) Applicant: Korea Atomic Energy Research Institute, Daejeon 305-353 (KR)
(72) Inventor: Byun, Myung-Woo, Jeongeup-si Jellabuk-do 580-050 (KR); Lee, Ju-Woon, Jeongeup-si Jeollabuk-do 580-050 (KR); Choi, Jong-II, Yeongdeungpo-gu Seoul 150-782 (KR); Kim, Jae-Hun, Jeongeup-si Jeollabuk-do 580-753 (KR); Song, Beom-Seok, Jungnang-gu Seoul (KR)
(74) Representative: Schweitzer, Klaus

(57) **Abstract**

Disclosed is an irradiation method which includes addition of vitamin C during sterilization of bone restorative demineralized bone matrix (DBM) with irradiation, so as to inhibit reduction of physical properties of a carrier containing DBM caused by irradiation and protect DBM formable bone morphogenetic protein (BMP) from irradiation. The method according to the present invention can provide bone restorative materials with more excellent stability and effectively controlled modification of physical properties by employing a sterilization process accompanied with addition of vitamin C during irradiation.

## Description

### BACKGROUND OF THE INVENTION

The present invention relates to a method of irradiation using a process of adding vitamin C, and more particularly, to an irradiation method including addition of vitamin C during sterilization of bone restorative demineralized bone matrix (hereinafter, often referred to as "bone matrix" or "DBM") with irradiation, so that it can inhibit reduction of physical properties of a carrier containing DBM caused by irradiation and protect DBM formable bone morphogenetic protein (hereinafter, often referred to as "BMP") from irradiation.

Autograft is on the decrease, whereas the market of bone graft substitutes such as DBM is extended as time goes on. In order to accommodate convenience of medical procedures manipulating such substitutes for bone repairing, the bone restorative material is typically combined with any desirable carrier to be used.

In order to use bone restorative carriers in bone restoration, high viscosity sufficient to retain a certain shape as well as bio-synthesis in the body are required. Thus, the above carrier may include any specific polymers, for example, MPEG-polyester, chitosan, small intestinal submucosa tissues and/or carboxymethyl cellulose and the like.

Naturally derived bones generally include organic and inorganic materials. The organic materials comprise growth factors, cartilage tissues, collagen and other proteins. The inorganic materials of the bone comprise non-stoichiometrically poorly crystalline apatitic (PCA) calcium phosphate with Ca/P ratio of 1.45 to 1.75, as described in Besic et al., J. Dental Res. 48(1):131, 1969. Mineral ingredients contained in the inorganic materials of the bone are continuously re-absorbed and reproduced by osteoclast and osteoblast in body.

Bone implant is often used to improve natural regeneration of the bone which has defects or injuries. Ideal bone implant must have bio-compatibility, be morphogenetic, that is, osteo-conductive and osteo-inductive at the same time, easily manipulated by a surgeon prior to transplantation, and retain inherent strength and properties in the body after transplantation.

Preferable one of the materials described above is organic bone-derivable material. Generally known bone-derivable materials include demineralized bone matrix (DBM) and recombined human bone morphogenetic proteins (rh-BMPs). For example refer to: US Patent No. 6,030,635; EP Application No. 0 419 275; International applications PCT/US00/03024, PCT/US99/01677 and PCT/US98/04904, etc.

Such organic bone-derivable materials are normally delivered to graft sites together with liquid or gelatin carriers. For example refer to: US Patent Nos. 6,030,635; 5,290,558; 5,073,373; and International application PCT/US98/04904, etc. Ideally used bone implant includes plenty of bone-derivable materials in order to greatly improve the regeneration ability thereof.

BMP belongs to TGFb-super family proteins. As a result of introducing demineralized bone matrix into muscle of a rat, ectopic bone formation was monitored at sites of the muscle containing the bone matrix. From the experiment, it was demonstrated that the bone matrix should contain any material to induce differentiation of undifferentiated cells among cell groups to form the bone in the bone matrix, thereby growing the bone. Such material contained in the bone matrix was a protein ingredient and called "bone morphogenetic protein." For example refer to Urist, MR, Strates, BS, bone morphogenetic protein. J. Dental Res. 50:1392-1406, 1971.

Bone morphogenetic proteins are a differentiation factor and were extracted on grounds of ability to induce the bone formation, as described in Wozney, JM, Science 242:1528-1534, 1988. Such protein produces a BMP family with at least thirty (30) constitutional members belonging to TGFb-super family proteins. The BMP family is classified into sub families including, for example: BMPs such as BMP-2 and BMP-4; osteogenetic proteins (Ops) such as OP-1 or BMP-7, OP-2 or BMP-8, BMP-5, BMP-6 and/or Vgr-1; cartilage derived morphogenetic proteins (CDMPs) such as CDMP-1 or BMP-14 and/or GDF-5; growth/differentiation factors (GDFs) such as GDF-1, GDF-3, GDF-8, GDF-11 or GDF-12 and GDF-14; and other sub families including BMP-3 or osteogenin, BMP-9 or GDF-2, and BMP-10.

Under this circumstance, there is a requirement for an improved technique to safely produce and manage bone restorative materials that can preserve or store such bone implant materials or bone graft substitutes, especially, DBM which contains bone tissue regeneration derivable materials, for long term *even in vitro* or *ex vivo* while maintaining inherent functions thereof. More particularly, it needs to introduce a production technique that conforms to a quality assurance program according to International Standards and Regulation Systems in order to facilitate export of biocompatible tissues such as the bone restorative materials.

Among microorganisms contaminating bio tissues such as the bone restorative material, viruses are very difficult to be removed and, especially, HIV, SV40, para influenza and herpes virus which often do significant harm to human body must be eliminated. Accordingly, lots of known chemicals have been commonly used to remove such organic contaminants. However, since residue remaining after sterilization sometimes causes undesirable side effects including, for example, dermatitis, tissue necrosis, etc. after transplantation, we still indeed demand for a safe sterilization method.

Irradiation techniques have been internationally approved for superiority and safety thereof which are sufficient to allow the techniques to be used in hygienic treatment of public health products. Thus, studies for practical use of the irradiation techniques are now actively in progress. For example, as a strong and effective method to establish safe production systems of bio-tissues such as bone restorative materials, there is still a significant requirement to develop a method of appropriately removing organic contaminants and/or contaminated organic materials using irradiation techniques.

According to International Standard/Technical Report ISO/TR 13409, it was demonstrated that small or rarely used tools could be sterilized or hygienically treated using the irradiation method with radiation dose of 25kGy. But, such high radiation dose may seriously affect physical properties of bone restorative materials and carriers while removing contaminants, that is, bacteria. For example, the irradiation with high radiation dose has problems such as reduction of viscosity of polymer based carriers and/or modification of biological and physical properties of bone restorative materials.

### SUMMARY OF THE INVENTION

Accordingly, the present invention is directed to solve the problems of conventional techniques as described above and, an object of the present invention is to provide an improved irradiation method that can inhibit reduction of physical properties of a carrier containing demineralized bone matrix (DBM) during the irradiation and protect DBM formable bone morphogenetic protein (BMP) from the irradiation.

In order to accomplish the above object, a preferred embodiment of the present invention provides an irradiation method which includes addition of vitamin C while sterilizing the bone restorative DBM and the carrier containing DBM with irradiation, so as to inhibit reduction of physical properties of DBM and the carrier caused by the irradiation and protect BMP from the irradiation.

DBM is known to have superior performance of regenerating bone and be useful for manufacturing bone restorative implants, bone growth accelerating compositions, and/or health aids or supplementary food products. DBM used in the irradiation method according to the present invention can be produced using DBM derived from mammals and widely known methods and/or techniques. For example refer to Russell et al., Orthopedics, 22(5)524-531, 1999.

Irradiation adopted in the present invention commonly uses at least one selected from a group consisting of gamma (γ)-ray, electron beam and X-ray and, preferably, uses gamma (γ)-ray in view of improvement of the performance for releasing BMP.

Absorption dose of the irradiation ranges from 2.5 to 100kGy and, preferably, from 20 to 50kGy. When the absorption dose is less than 2.5kGy, a desirable purpose of sterilization by the irradiation is not achieved while there may be problems such as decomposition of materials caused by high dose of radiation, in case that the dose exceeds 100kGy.

Irradiation may include irradiation to a composite containing the bone matrix combined with bone restorative carrier.

The above bone restorative carrier includes at least one selected from, for example, carboxymethyl cellulose, chitosan, fibrins and small intestinal submucosa. On the ground that activity for bone morphogenesis is increased during allograft, preferred is carboxymethyl cellulose.

In case of the irradiation to the composite containing the bone matrix combined with the bone restorative carrier, the composite is preferably prepared by combining the bone matrix with the bone restorative carrier in a relative ratio ranging from 8:2 to 6:4. The reason is that, in order to use the bone matrix as bio-material for accelerating bone regeneration, the bone matrix should be used as the composite with the bone restorative carrier which is in the form of polymeric gel such as carboxymethyl cellulose in consideration of easier allograft.

The irradiation generates free radical groups such as OH radicals in cells, which cause polymer chains to be cut and/or alteration of protein structure, in turn, protein denaturation. In case that organisms or cells are exposed to radiation, water molecules become ionized then cause radiolysis as follows.

When water is ionized, an electron is released from a water molecule (as shown in Reaction Scheme 1) and the released electron is absorbed by another water molecule (Reaction Scheme 2).

**H₂O→H₂O⁻ + e_{aq}⁻** (1)

**e_{aq}⁻ + H₂O →H₂O⁻** | (2)

By the reaction steps as described above, cationic water molecules H₂O⁺ and anionic water molecules H₂O⁻ are generated and such ions become decomposed in the presence of other water molecules so as to form ions and free radicals (Reaction Scheme 3).

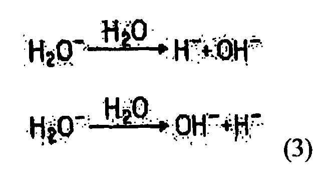

Even though H⁺ ions and OH ions have not so great energy, primary free radicals such as e_{eq}⁻, H and OH created by radiolysis of the water molecules have high reactivity such that they bring about secondary reaction at sites at which the primary free radicals were created. Moreover, secondary free radicals generated from the secondary reaction include, for example, HO₂⁻ and O₂⁻, which have relatively weaker reactivity than that of the first free radicals such that they may be transferred so far where they interact with constitutional ingredients of the body.

In purified water, the above free radicals react together to generate H₂, H₂O, H₂O₂ etc. (Reaction Schemes 4 to 6).

H + OH → H₂O | (4)

H + H → H₂ (5)

OH + OH →H₂O₂ | (6)

Herein, H₂O₂ is eliminated by enzymes such as peroxidase and catalase in the body, while O₂⁻ is removed by superoxide dismutase (SOD). Such ionization is not restricted to only the water molecules but also applied to a variety of organic materials in cells and generates peroxides based on the same principle so that the generated peroxides react with protein and/or other ingredients, thereby causing metabolic disorders.

As described above, the process by which water molecules contained in cells absorb radiation energy and generate free radicals which react with target cells to cause biological variation, is generally designated as indirect reaction of the irradiation.

Materials with radical removal effect include and are classified into enzymes, fat-soluble or lipophilic compounds, water-soluble compounds and polymeric antioxidant materials. Enzymes are not limited but include SOD, GSH (glutathione peroxidase) and the like. Fat-soluble compounds include vitamin E and beta-carotene.

Water-soluble compounds comprise, for example, vitamin C while albumin being in the polymeric antioxidants. Among them, vitamin C shows less adverse effect to the body even taken in large amounts, and a great quantity of vitamin C is easily and commercially available in the market. Vitamin C is one of the water-soluble antioxidants, which rapidly reacts with peroxides or free radicals, removes the radicals and increases activity of anti-oxidizing enzymes to exhibit anti-oxidative effect. It has been well known that intake of vitamin C inhibits formation of nitrosamine from nitrates and, for animal experiments, can reduce carcinogenic properties of nitrite.

If 10g of vitamin C is administered to patients with cancer, the survival rate of the patients increases by 4 times more than a control. Also, vitamin C can inhibit mutation of microorganisms caused by nitro compounds, derive secretion of estrogen in mammals including humans and prevent mutagen precursors from being combined with DNA.

In the irradiation of the present invention, concentration of vitamin C added preferably ranges from 10ppm to 2%. If less than 10ppm, the purpose of adding vitamin C cannot be achieved. On the other hand, in the case of exceeding 2%, it may cause problems due to low pH values.

BMP is advantageously used in production of bone restorative implants, bone growth accelerating compositions, and/or health aids or supplementary food products, but not limited thereto so far as the products have bone morphogenesis derivable performance.

For example, BMP preferably includes BMP-2, BMP-7 and the like. BMP-2, that is, bone morphogenetic protein-2 strongly derives autologous and heterologous bone morphogenesis *in vivo* and, in addition to, is widely known as an effective bone morphogenetic derivative to differentiate preosteoblast or undifferentiated stem cells into osteoblast *in vitro.* Further, as ectopic bone is formed when BMP-2 is intramuscularly introduced *in vivo,* it was found that, if BMP-2 is introduced into C2C12 cells which are mouse premyoblastic cell lines, such cells stop differentiation into muscle cells and express marker genes of osteoblast.

It is well known that BMP-7 is a material relating to bone morphogenesis and has an important role in forming teeth and eyes during development. Moreover, it was disclosed that BMP-7 cannot be generated in body of an adult person. For example refer to Dev. Biol. 207(1): 176-188, 1999.

### DETAILED DESCRIPTION OF THE INVENTION

Hereinafter, the present invention will become apparent from the following examples with reference to the accompanying drawings. However, the examples are intended to illustrate the invention as preferred embodiments of the present invention and do not limit the scope of the present invention.

### EXAMPLE 1

### Prevention of viscosity reduction caused by irradiation of carrier carboxymethyl cellulose by addition of vitamin C

In this example, variation of viscosity of 3% carrier carboxymethyl cellulose gel by addition of vitamin C was determined after the gel was irradiated.

The irradiation was performed by using 100,000 Ci of radiation source, Co-60 gamma (γ)-ray irradiation facility in the Advanced Radiation Technology Institution, Korea Atomic Energy Research Institute (KAERI) with radiation dose of 10kGy per hour at room temperature of 12±1°C. γ-ray radiation dose emitted to the carrier was regulated to attain overall absorption dose of 30kGy. The absorption dose was determined by means of ceric-cerous dosimeter with relative error of ±0.2kGy.

It was identified from the following Table 1 that reduction of viscosity due to γ-ray irradiation could be inhibited by adding vitamin C to the carrier carboxymethyl cellulose on basis of concentration of vitamin C.

**TABLE 1: Comparison of viscosities of carboxymethyl cellulose based on concentration of vitamin C after γ-ray irradiation**

| SAMPLE | Radiation dose of γ-ray (kGy) | Concentration of vitamin C (wt.%) | Viscosity (cp %) |
|---|---|---|---|
| 3% carboxymethyl cellulose | 0 | 0 | 100 |
| 3% carboxymethyl cellulose | 0 | 1 | 101.3 |
| 3% carboxymethyl cellulose | 30 | 0 | 4.76 |
| 3% carboxymethyl cellulose | 30 | 0.1 | 44.76 |
| 3% carboxymethyl cellulose | 30 | 1 | 58.7 |

As shown in the above Table 1, the viscosity was not much increased even when vitamin C was added in amount of 1% to the carboxymethyl cellulose gel. However, it was found that 30kGy of γ-ray irradiation with vitamin C greatly increased the viscosity of carboxymethyl cellulose gel, compared with a control without addition of vitamin C.

Furthermore, the viscosity was increased as the concentration of vitamin C was increased. More particularly, in case of adding 0.1% of vitamin C, the viscosity increased by more than 9 times, in comparison with the control without addition of vitamin C after γ-ray irradiation. Alternatively, adding 1% of vitamin C increased the viscosity by more than 12 times, compared with the control without addition of vitamin C after γ-ray irradiation.

With regard to a sterilization process of carrier containing bone restorative material by using electron beam, influence of adding vitamin C to inhibition of reduction physical properties of the carrier was identified.

The electron beam irradiation of carboxymethyl cellulose was carried out using a linear electron accelerator of the Advanced Radiation Technology Institution in Jeongeup city, Korea Atomic Energy Research Institute (KAERI). Such accelerator was UELV-10-10S model with electron beam energy of 10MeV and current of 1mA manufactured by NIIEFA, which had an inspection window with distance of 200mm and dimension of 8×20mm. Absorption dose of the electron beam was determined from current value and radiation dose measured. The radiation dose used was 30kGy.

From the following Table 2, it was demonstrated that reduction of viscosity caused by the irradiation of electron beam could be inhibited dependent on concentration of vitamin C added to the carrier carboxymethyl cellulose.

**TABLE 2: Comparison of viscosities of carboxymethyl cellulose based on concentration of vitamin C after electron beam irradiation**

| SAMPLE | Radiation dose of electron beam (kGy) | Concentration of vitamin C (wt.%) | Viscosity (cp%) |
|---|---|---|---|
| 3% carboxymethyl cellulose | 0 | 0 | 100 |
| 3% carboxymethyl cellulose | 0 | 1 | 101.3 |
| 3% carboxymethyl cellulose | 30 | 0 | 40 |
| 3% carboxymethyl cellulose | 30 | 0.1 | 43.5 |
| 3% carboxymethyl cellulose | 30 | 1 | 64.4 |

As shown in the above Table 2, the viscosity was not much increased even when vitamin C was added in amount of 1% to the carboxymethyl cellulose gel. However, it was found that 30kGy of electron beam irradiation increased the viscosity of carboxymethyl cellulose gel, compared with a control without addition of vitamin C.

Furthermore, the viscosity was increased as the concentration of vitamin C was increased. More particularly, in case of adding 0.1% of vitamin C, the viscosity increased by more than 8.8%, in comparison with the control without addition of vitamin C after electron beam irradiation. Alternatively, adding 1% of vitamin C increased the viscosity by more than 61 %, compared with the control without addition of vitamin C after electron beam irradiation.

### EXAMPLE 2

### After irradiation for sterilizing carrier containing bone restorative DBM. inhibition of biological modification of BMP contained in DBM by addition of vitamin C

In this example, when the irradiation was applied to sterilize the carrier including bone restorative DBM, the irradiation was practically carried out with addition of vitamin C to inhibit denaturation of BMP contained in DBM.

For quantification of BMP extracted from DBM, osteoinductivity of the bone matrix by BMP was quantified with ALP assays directly using C2C12 cells. This assay will be described in detail below.

First, C2C12 cells were added at 5×10⁴ cells/well to 24-well plate. 4 hours after adding the cells to the 24-well plate, the media was changed to 1% FBS media and a transwell was placed in the 24-well plate to treat 100mg of the demineralized bone matrix while introducing 1ml of the media thereto.

After culturing for 48 hours, the media were discarded and the cultured cells were rinsed out twice with cold PBS (phosphate buffered saline). Subsequently, 0.5% triton-100/PBS was added in amount of about 500µℓ to 1mℓ into the wells and left for 1 to 2 minutes. Then, a scraper was used to scratch the cells off the wells and a freezing/thawing process, that is, lyophilization was repeated three times to break cell membrane.

After dilution in series, the samples were placed into the plates in amount of 50 µℓ per plate. Only the enzyme buffer was introduced in blank of each of the plates, 50 µℓ of pNPP (para-nitrophenyl phosphate) substrate solution was added thereto, and the sample was cultured at room temperature for 10 to 20 minutes.

Finally, after 50µℓ of stop solution was added to the cultured sample and rapidly agitated to blend it, absorbency of the sample was detected at 405nm. An assay buffer was used as standard for the detection, diluted in series and detected at 405nm as was the sample.

Measured values from the experiments were subjected to ANOVA (analysis of variance) using SPSS software and, if they passed the significance test, a significant difference between least square mean values was identified using Duncan's multiple range tests (p < 0.05).

With regard to the γ-ray irradiation for sterilizing a mixture of the carrier carboxymethyl cellulose and the bone restorative DBM, it was demonstrated from the following Table 3 that denaturation of BMP contained in DBM could be inhibited by addition of vitamin C to the mixture.

**TABLE 3: Comparison of activities of BMP contained in bone restorative DBM based on concentration of vitamin C, after γ-ray irradiation for sterilizing the bone restorative DBM**

| SAMPLE | Radiation dose of γ-ray (kGy) | Concentration of vitamin C (wt.%) | 1×ALP concentration (pmoles) % |
|---|---|---|---|
| DBM+3% carboxymethyl cellulose | 0 | 1 | 100 |
| DBM+3% carboxymethyl cellulose | 30 | 0 | 43.23 |
| DBM+3% carboxymethyl cellulose | 30 | 0.1 | 74.57 |

As shown in the above Table 3, when the mixture of DBM and 3% carboxymethyl cellulose underwent the γ-ray irradiation with radiation dose of 30kGy for sterilizing the mixture, the results of ALP assay demonstrated that activity of BMP was reduced to 43%. On the other hand, in the γ-ray irradiation with radiation dose of 30kGy, addition of 0.1% of vitamin C to the mixture increased activity of BMP by 72%, compared with the control without addition of vitamin C.

With regard to the electron beam irradiation for sterilizing a mixture of the carrier carboxymethyl cellulose and the bone restorative DBM, it was demonstrated from the following Table 4 that denaturation of BMP contained in DBM could be inhibited by addition of vitamin C to the mixture.

**TABLE 4: Comparison of activities of BMP contained in bone restorative DBM based on concentration of vitamin C, after electron beam irradiation for sterilizing the bone restorative DBM**

| SAMPLE | Radiation dose of electron beam (kGy) | Concentration of vitamin C (wt.%) | 1×ALP concentration (pmoles) % |
|---|---|---|---|
| DBM + 3% carboxymethyl cellulose | 0 | 1 | 100 |
| DBM + 3% carboxymethyl cellulose | 30 | 0 | 88.33 |
| DBM + 3% carboxymethyl cellulose | 30 | 0.1 | 138.50 |

As shown in the above Table 4, when the mixture of DBM and 3% carboxymethyl cellulose underwent the electron beam irradiation with radiation dose of 30kGy for sterilizing the mixture, the results of ALP assay demonstrated that activity of BMP was reduced to 88%. On the other hand, in the electron beam irradiation with radiation dose of 30kGy, addition of 0.1% of vitamin C to the mixture increased activity of BMP by 57%, compared with the control without addition of vitamin C.

Consequently, the method according to the present invention is effective to produce bone restorative materials with improved stability and efficiently controlled modification of physical properties by a sterilization process accompanied with addition of vitamin C during irradiation.

The bone restorative DBM and the carrier containing the same produced after the irradiation according to the present invention may be advantageously used in production of bone restorative implants, bone growth accelerating compositions, and/or health aids or supplementary food products.

It is understood that various other modifications and variations will be apparent to and can be readily made by those skilled in the art without departing from the scope and spirit of the present invention as defined by the appended claims.

## Claims

1. An irradiation method including addition of vitamin C during sterilization of bone restorative demineralized bone matrix (DBM) and bone restorative carrier containing DBM with irradiation, so that it can inhibit reduction of physical properties of the carrier caused by the irradiation and protect DBM formable bone morphogenetic protein (BMP) from the irradiation.

2. The method according to Claim 1, wherein the irradiation is conducted by at least one selected from a group consisting of gamma (y) -ray, electron beam and X-ray.

3. The method according to Claim 1, wherein absorption dose of the irradiation is in the range of from 2.5 to 100kGy.

4. The method according to Claim 3, wherein absorption dose of the irradiation is in the range of from 10 to 50kGy.

5. The method according to Claim 1, wherein the bone restorative carrier comprises at least one selected from a group consisting of carboxymethyl cellulose, chitosan, fibrins and small intestinal submucosa.

6. The method according to Claim 1, wherein concentration of vitamin C added ranges from 10ppm to 2%.

7. The method according to Claim 1, wherein the bone morphogenetic protein is BMP-2 or BMP-7.

8. An implantable bone growth inducing composition comprising demineralized bone matrix (DBM), bone restorative carrier and vitamin C in an amount of 10ppm to 2%, wherein said composition has been sterilized using gamma-ray, electron beam or X-ray at an irradiation absorption dose of from 10 to 50kGy.

9. The composition of claim 8, wherein the bone restorative carrier comprises at least one carrier selected from a group consisting of carboxymethyl cellulose, chitosan, fibrins and small intestinal submucosa.

10. The composition of claim 8, wherein the demineralized bone matrix comprises bone morphogenetic protein of type BMP-2 or BMP-7.
